# EUROPEAN PATENT APPLICATION

(11) **EP 1 174 129 A1**
(43) Date of publication of application: **23.01.2002**
(21) Application number: 00114909.5
(22) Date of filing: 17.07.2000
(51) Int. Cl.: A61K 31/00, A61K 31/4439, A61P 35/00

(54) **Use of a matrix-metalloprotease inhibitor for the treatment of cancer**

(71) Applicant: Zenner, Hans Peter, Prof. Dr. med., 72070 Tübingen (DE); Simon, Christian, Dr., 72076 Tübingen (DE)
(72) Inventor: Zenner, Hans Peter, Prof. Dr. med., 72070 Tübingen (DE); Simon, Christian, Dr., 72076 Tübingen (DE)
(74) Representative: Patentanwälte Ruff, Wilhelm, Beier, Dauster & Partner

(57) **Abstract**

The invention relates to the use of an active agent, particularly an inhibitor, in treating cancer by influencing, preferably inhibiting the expression of matrix-metalloproteases. The targets for this active agent can especially relate to downstream regulators of the matrix-metalloprotease 9 (MMP-9) signal transduction pathway. According to the invention, expecially inhibitors of p38beta and p38gamma as also of mitogen-activated kinase kinase 6 (MKK6) and mitogen-activated kinase kinase 3 (MKK3) can be applied.

## Description

The invention relates to the use of an active agent, particularly an inhibitor, of matrix-metalloproteases expression. More specificly, this invention relates to the use of such agents in connection with the treatment of cancer, especially cancer invasion.

As is known, the degradation of the extracellular matrix is a very complex process and it is part of many pathological and physiological processes. Thereby, the proteolytic degradation of the extracellular matrix plays a crucial role in cancer invasion as also in non-neoplastic tissue remodelling processes. The invasive phenotype of cancer critically depends on the activity and expression of several proteases. The role of the matrix-metalloprotease enzymes in this tumor cell-mediated extracellular matrix proteolysis is well established. One of these matrix-metalloproteases is the MR 92,000 type IV collagenase (MMP-9). MMP-9 degradates the basement membrane, a structure that is largely composed of type IV collagen and which normally separates the epithelial from the stromal compartment (1, 2). Many growth factors induce expression of MMP-9 and other proteases by binding to transmembrane tyrosine receptors which in turn activate signal transduction pathways (3, 4). However, some cell lines produce large amounts of proteases even in the absence of such growth factors, suggesting a constitutive activation of signaling cascades as one underlying mechanism (5). In fact, constitutive activation of signal transduction pathways has been described for renal cell carcinomas, leukemia's as well as numerous other cancers. In connection with these constitutive activation of signal transduction pathways reference is made to the publications of H. Oka et al. and to S.C. Kim et al. (6, 7).

It is well known by those who are skilled in the art that the stress- and mitogen-activated protein kinases (SAPK and MAPK) play a central role in signaling pathways. There are three major subfamilies including p38/RK, JNK/SAPK, and p42/p44 MAPK's/ERK's. In general ERK's are stimulated by mitogens and differentiative factors, while JNK and p38 are activated by environmental stress such as ultraviolet light, osmotic stress but also inflammatory cytokines. All three subfamilies regulate apoptosis, ERK's are negative but JNK's and p38's are positive regulators (8). So far four human isoforms of p38 have been cloned: p38alpha (9), p38beta (10), p38gamma (11), which also has been termed SAPK3 or ERK6 and p38delta (12), also termed SAPK4. The alpha- and beta-isoforms are predominantly involved in mediating proinflammatory signals to the nucleus and regulate apoptosis (8). p38gamma has been implicated to play a role in muscle development and response to hypoxic stress (13, 14). p38alpha and -beta are widely distributed in human tissues, their expression was found to be most abundant in brain and heart (10). SAPK3 is predominantly present in skeletal muscle (15, 16). Little is known about the function of SAPK4. High levels of expression were found in salivary, pituitary and adrenal gland tissue (12).

Important upstream regulators of p38 isoforms include the protein kinases MKK6 and MKK3.

Findings and investigations up to now concerning the involvement of matrix-metalloproteases in cancer invasion and metastasis have focused on the functions of the various matrix-metalloproteases enzyme domains and their interactions with inhibitor domains. For instance it is known that the proteolytic activity of the matrix-metalloproteases involved in extracellular matrix degradation must be precisely regulated by their endogenous protein inhibitors, the tissue inhibitors of metalloproteases (TIMPs). These tissue inhibitors of metalloproteases play also an important role in matrix degradation by tumor cells. The activity of TIMP and MMP was analysed in several carcinomas, for instance in renal cell carcinoma as also in gastric cancer. In connection with these studies reference is made to the publications of A. Kugler and G.I. Murray et al. (17, 18). These tissue inhibitors of metalloproteases are a family of secreted proteins that play a crucial role in the regulation of the activity of the secreted metalloproteases. Up to now three of them are characterized (TIMP1, TIMP2 and TIMP3). They influence the activation of the prometalloproteases and act to modulate proteolysis of extracellular matrix, notably during tissue remodelling and inflammatory processes. A characterization of these tissue inhibitors of matrix-metalloproteases, appears in the publication of D.T. Denhardt et al. (19). There are also synthetic matrix-metalloproteases inhibitors like marimastat (BB-2516), a butanediamid-derivative with the IC₅₀ value in the micromolar range. C. Simon et al. (20) demonstrated that the enhanced MMP-9 secretion and in-vitro invasiveness in a human squamous cell carcinoma cell line (UM-SCC-1) after treatment with phorbol myristate acetate (PMA), a known tumor promoter widely used in the study of skin carcinogenesis (reviewed in (21)), could be inhibited by using the general p38 inhibitor SB 203580.

It has now surprisingly been found that the constitutive activity of the p38 as also the MKK6 and/or MKK3 pathway plays a crucial role in the high-level expression of MMP-9 in cancer cells. As a result of these unexpected findings, the use of active agents which are targeted/directed against downstream regulators of MMP-9 expression for the treatment of cancer, especially cancer invasion is made possible.

Thus, the problem of the invention of making available active agents for the treatment of cancer, is solved by the use according to claims 1 and 2. Prefered embodiments are given in the dependent claim 3 to 17. The content of all these claims is hereby incorporated into the description by reference.

According to the invention, at least one active agent is used for influencing, particulary inhibiting the expression of matrix-metalloproteases in eukaryotic cells for the treatment of cancer. This in particular also covers the use of such active agent for producing a corresponding medicament or a corresponding pharamaceutical composition. According to the present invention, the active agent can optionally be used in the form of its pharmaceutically acceptable salts and optionally together with a pharmaceutically acceptable carrier.

The active agents used according to the present invention are those which preferably influence, particulary inhibit the above-mentioned matrix-metalloproteases involved with cancer, preferably cancer invasion. According to the invention one prefered matrix-metalloprotease involved in cancer invasion is the matrix-metalloprotease 9.

In one prefered embodiment of this invention the active agent used is preferably targeted against at least one member of matrix-metalloprotease signal transduction pathways, particularly against one member of the MMP-9 signal transduction pathway. One prefered member of this MMP-9 signal transduction pathway is the so-called p38 protein family. In the use according to this invention one of these p38 proteins is the p38beta protein, another prefered member according to the use of the present invention is the p38gamma (SAPK3 or PRK6) protein. Another prefered target for the active agent according to the invention is the mitogen-activated kinase kinase family. Two prefered members of this mitogen-activated kinase kinase family are the mitogen-activated kinase kinase 6 (MKK6) and the mitogen-activated kinase kinase 3 (MKK3). In the use according to the invention one member of the MMP-9 signal transduction pathway can be targeted alone by the active agent, but there can also be a random combination of two or three or even more different members of the MMP-9 signal transduction pathway which are targeted by the active agent.

In another prefered embodiment it is optionally also possible that an activator, regulator and/or a biological precursor of the matrix-metalloprotease signal transduction pathway, preferably of the MMP-9 signal transduction pathway, is targeted and/or influenced by the active agent. These activators, regulators and/or biological precursors may be e.g kinases which are known to be involved in the regulation of the enzymatic activity of proteases, transcriptional factors which are responsible for the expression level of proteases, proteases which are responsible for the activation of prometalloproteases or tissue inhibitors, or even up to date unknown compounds which can be influenced by the active agent.

According to the invention it is possible to use known or also novel active agents. In one prefered embodiment of the invention the active agent is a compound with specific inhibitory capacity against at least one member of the matrix-metalloprotease signal transduction pathway, preferably against the MMP-9 signal transduction pathway. This active agent is preferably a comparably small molecule of low molecular weight (MW), especially with a MW < 1000. It is further preferred, if such active agent is an imidazole derivative. Such imidazole derivatives, like SB 203580 (MW 377,4) or SB 202190 (MW 331,3) which are both obtainable from Calbiochem, San Diego, Ca, USA, are known to be potent inhibitors of kinase expression. In another prefered embodiment of this invention, the active agent is an inhibitor of p38 proteins. This can be a known or also a further novel inhibitor of p38 proteins. In another prefered embodiment of this invention, the active agent is an inhibitor of the mitogen-activated kinase kinase family. This inhibitor can be a peptide inhibitor of the mitogen-activated kinase kinase family like the kinase dead mutants constructed with standard molecular biology techniques as used in this description or also a novel inhibitor compound. Several inhibitors are known and one can find a few of them in the publications of Y. Fukami et al., J.C. Lee et al., and D. Fabbro et al. (22-24).

In another prefered embodiment according to the present invention the active agent is an inhibitor of activators, regulators and/or biological precursors of the matrix-metalloprotease signal transduction pathway, which might be kinase inhibitors, transcription factors inhibitors, tissue inhibitors, proteases inhibitors and other known or novel inhibitors of the matrix-metalloprotease signal transduction pathway.

In another prefered embodiment according to the invention the active agent is a polynucleotide which encodes a peptide or a polypeptide that inhibits the expression of matrix-metalloproteases, preferably inhibits p38 and/or mitogen-activated kinase kinase activity. This peptide can be e.g. a p38 kinase deficient mutants, a mitogen-activated kinase kinase dead mutant and other peptides known to those who are skilled in the art.

The invention can be used for treatment of all kinds of cancer, expecially cancer with a overexpression of matrix-metalloproteases and therefore with a high invasiveness and metastasis of this cancer. A overexpression of MMP-9 was reported in squamous epithelial carcinomas of the head, neck, skin and stomach as also in fibrosarcomas of the stomach. An increased MMP-9 level was also found in the serum of patients with colon-, breast- and hepatocellular carcinomas. Therefore, among the treatable illnesses particular reference is made to the above noted cancers. As is generally known metastatic disease (but also often invasive tumor growth itself) limits the survival of cancer patients. The reasons of the constitutive activation of signal transduction pathways in cancer are up to now unknown. It might result from mutations of growth factor receptor genes such as gene amplifications or autocrine loops, i.e. expression of ligand and receptor in the same tissue. The above mentioned cancers are good targets for the active agent according to invention as cancer invasion is a vexing problem in these cancers.

According to the invention it is possible to select the administration form of the active agent. This form can be adapted to the age, sex or other characteristics of the patient, the severity of the cancer and other parameters. Conventional pharmaceutical carriers, diluents or conventional additives can be present.

The dosage can be freely selected as a function of the clinical picture and the condition of the patient.

The invention finally comprises a pharmaceutical composition or a medicament for the treatment of cancer, which contains at least one active agent for influencing, particulary inhibiting the expression of matrix-metalloproteases in eukaryotic cells. Relating to the individual features of such composition or medicament, reference is made to the corresponding description text above.

The described features and further features of the invention can be gathered from the following description of prefered embodiments in conjunction with the subclaims. The individual features can be implemented separately or in the form of subcombinations.

### MATERIALS AND METHODS

Tissue Culture and Materials. UM-SCC-1 cells (known to a skilled person and obtainable from Dr. Thomas Carey, University of Michigan, Ann Arbor, MI), Hlac82 (known to a skilled person and obtainable from Dr. Hans Peter Zenner, University of Tübingen, Germany) and NIH 3T3 cells (maintained by nearly every cell biology laboratory and also obtainable from Dr. Hans Peter Zenner, see above), were maintained in McCoy's 5A culture medium supplemented with 10% fetal bovine serum (FBS, Gibco Life Technologies, Karlsruhe, Germany). For the collection of conditioned medium for zymography and Western blotting, 80% confluent UM-SCC-1, Hla82 and NIH 3T3 cells respectively were incubated in serum-free medium (McCoy's 5A medium, components known to a skilled person and available from Gibco Life Technologies, Karlsruhe, Germany) for 48 hours, when indicated with or without SB 203580 (Calbiochem, San Diego, CA) or carrier (DMSO) added at the same time. In the following "serum-free medium" will also be abbreviated as "SFM". The culture medium was collected and proliferation determined after incubating cells in 0.2-mg/ml MTT-vital stain and reading aliquots of DMSO dissolved formazan crystals by spectrophotometry at 570 nm. Growth curves were constructed as described (25) using various amounts of SB 203580 added at the same time after allowing 12 hours for cell attachment.

Zymography. Zymography was performed exactly as described (20, 25) using SDS-PAGE gel containing 0.1% (wt/vol) gelatin to assay for MMP-9. MMP-dependent proteolyses was detected as white zones in a dark field.

Western Blotting. For the detection of MMP-9 in conditioned medium, medium from equal numbers of cells was denatured in the absence of reducing agent, proteins resolved by SDS-PAGE and then transferred to a nitrocellulose filter. The filter was blocked with 3% BSA and incubated with a mouse monoclonal antibody to matrix metalloprotease (#IM37L Oncogene Research Products, Calbiochem, Cambridge, MA). Subsequently, the blot was incubated with horse radish peroxidase-conjugated anti-rabbit IgG and immunoreactive bands visualized by ECL (Enhanced Chemiluminescence), a commercially available immunoblotting detecting system as described by the manufacturer (Amersham Life Science, Arlington Heights, IL). p38al-pha and SAPK3 protein was detected using monoclonal antibodies equally recognizing phospho- and dephospho-p38 (sc-535-G and sc-6023, Santa Cruz, Santa Cruz, CA). Briefly cells were extracted in RIPA-buffer containing PMSF (100mg/ml) and sodium orthovanadate (1 mM). SDS-PAGE was used to resolve proteins extracted under denaturing conditions. The filter was blocked with 3% BSA and subsequently incubated with the primary antibody over night. To visualize immunoreactive bands the ECL-system was again used.

In-gel Kinase Assay for p38alpha activity and SAPK3 activity assay. Kinase assays for p38alpha activity were performed as described (20). Briefly, cells were extracted with buffer A [1% NP40 (octylphenoxy polyethoxy ethanol), 25 mM Tris-HCl (pH 7.4), 25 mM NaCl, 1 mM sodium vanadate, 10 mM NaF, 10 nM sodium pyrophosphate, 10 nM okadaic acid, 0.5 mM EGTA, and 1 mM phenylmethylsulfonyl fluoride]. Extracted protein was incubated with 2 µg of the anti-p38alpha antibody immunoreactive with human and mouse p38alpha (sc-535-G, Santa Cruz, Santa Cruz, CA) and Protein-A agarose beads (2 mg) for immunoprecipitation. The beads were washed with buffer A and resuspended in 2X sample buffer, and the immune complexes were electrophoresed in a polyacrylamide gel containing myelin basic protein. The gel was treated sequentially with buffers containing 20% 2-propanol, 5 mM 2-mercaptoethanol, 6 M guanidine HCl and 0.04% Tween 40-5 mM 2-mercaptoethanol. The gel was then incubated at 25°C for 1 h with 10 µM ATP and 25 µCi of [³²P]ATP in a buffer containing 2 mM dithiothreitol-0.1 mM EGTA-5 mM MgCl₂, washed in a solution containing 5% trichloroacetic acid and 1% sodium pyrophosphate, dried, and autoradiographed. For SAPK3 activity cells were extracted in a buffer A, extracted protein incubated with 2 µg of the anti-SAPK3-antibody immunoreactive with human and mouse SAPK3 (06-603, Upstate Biotechnology, Lake Placid, NY, USA) and protein G agarose beads. Beads were washed in buffer A and kinase buffer (50mM HEPES, 0.1 mM EDTA, 0.0001% Brij35, 0.0001% β-mercaptoethanol, 150mM NaCL, 01mg/ml bovine serum albumin) and subjected to kinase reaction with 1 µg ATF2 (sc-4007, Santa Cruz, Santa Cruz, CA, USA) as the substrate in 40 µl of reaction buffer (kinase buffer, 0.3mM ATP, 0.4M MgCl₂) at 30°C for 30 min. The reaction was terminated by adding 2X reducing sample buffer and heating to 100°C for 5 min. The beads were removed by centrifugation. The supernatant was subjected to imunoblotting as described above with an anti-phospho-ATF2-antibody. Immunoreactive bands were visualized using the ECL-system.

In Vitro invasion assays. Invasion assays were performed as described (20, 25) using filters with 8 µm pore size coated with 1/3 diluted Matrigel®/SFM (Becton Dickinson, Bedford, MA). Cells were plated out in SFM containing SB 203580 or DMSO, the carrier of SB 203580, at similar concentrations. For experiments utilizing a mouse monoclonal MMP-9 Antibody (#IM09L, Oncogene Research Products, Cambridge, MA) (0.5, 1, and 10 µg/ml) cells were either plated out in SFM plus antibody of SFM plus similar amounts of preimmune serum. The amount of invasion was determined on the basis of the MTT activity on the lower side of the filter as a percentage of the total activity in the chamber.

Transient transfections with subsequent CAT-ELISA. Transient transfections were carried out using Lipofectamin ® (GIBCO, Life Technologies, Karlsruhe, Germany) for transient transfection as described by the manufacturer. UM-SCC-1 and NIH 3T3 cells were co-transfected at 70% confluence with a CAT reporter construct containing 670 bp of the MMP9 wild-type promoter including the transcriptional start site or the promoterless CAT construct (SV₀) (3µg) along with a pCDNA3-MKK-6 or -MKK-3 constitutive active mutant (0.03-3µg) as described (26), or dominant negative p38alpha, beta, SAPK3, SAPK4 or MKK-6 mutants with a one- or twofold molar excess to the promoter construct (kindly provided by Dr. J. Han, Scripps Research Institute, La Jolla, CA). The transfected DNA-amount was equalized in each sample using mock control vector (pCDNA3). CAT ELISA, measuring CAT protein expression, was performed according to the manufacturer (Roche Diagnostics, Mannheim, Germany).

In the drawings it is shown:
Figure 1: Influence of SB 203580 on MMP-9 expression (A), in-vitro invasion (B) and growth (C) of UM-SCC-1 cells.
Figure 2: Requirement of MMP-9 secretion for in-vitro invasion in different cell lines (A), expression of MMP-9 in different cell lines (B) and percentage of in-vitro invasion after incubation with anti-MMP-9 antibody (C).
Figure 3: Influencing of MMP-9 promoter activity after treatment with dominant negative p38 isoform proteins (A), and the expression of p38alpha and p38gamma in two different cell lines (B-E).
Figure 4: Expression of MMP-9 in two different cell lines (A), influencing of MMP-9 promoter activity after treatment with a kinase deficient MKK6 (B) and constitutive active MKK6 and MKK3 (C) mutants.

### Experiment 1

UM-SCC-1 cells were plated out in McCoy's 5A culture medium supplemented with 10 % fetal bovine serum (FBS, Gibco Life Technologies, Karlsruhe, Germany) and replenished the following day with serum-free medium containing SB 203580 (10µM, Calbiochem, San Diego, CA) or carrier (dimethylsulfoxide DMSO, 0.01 %). After 48 hours, the condition medium was harvested and proliferation rates were assayed with 3-(4,5-dimethylthiazol-2-yl)-2,5-diphenyltetrazolium bromide (MTT). Aliquots of condition medium, normalized for proliferation differences, were subjected to immunoblotting using a monoclonal anti-MMP-9 antibody. Subsequently, the blot was incubated with horse radish peroxidase-conjugated anti-rabbit IgG and immunoreactive bands visualized by ECL as described by the manufacturer (Amersham, Arlington Heights, IL). A reduction of protein expression of 70 % was noted according to densitometric measurement. The data are typical of triplicate experiments.

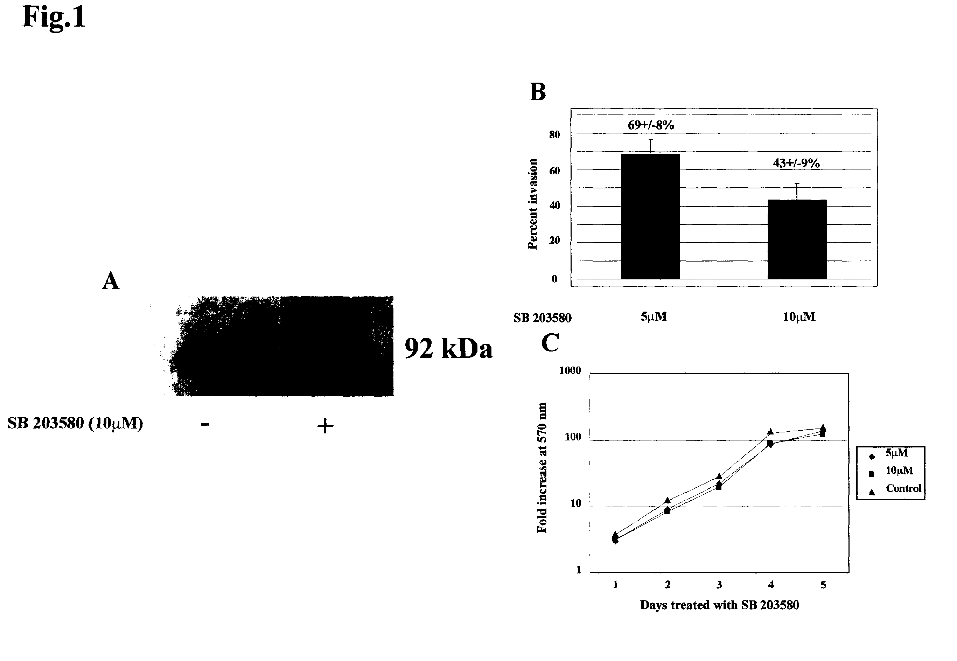

The results of experiment 1 are represented in Figure 1.

Figure 1A shows that the squamous cell carcinoma cell line UM-SCC-1 which constitutively produces large amounts of MMP-9 and displays an in-vitro and in-vivo invasive phenotype is influenced by treatment with the imidazol derivative SB 203580. SB 203580 reduced MMP-9 protein expression by approximately 70% at concentrations of 10µM, as evidenced by immunoblotting analysis.

In Figure 1B, UM-SCC-1 cells were plated out on filters precoated with Matrigel® in serum-free medium and incubated with various amounts of SB 203580 for 60 hours to assay for in-vitro invasion. The concentration of the carrier (DMSO) was maintained at 0.1%. Invasion is expressed as the percentage of cells invading through the Matrigel®. Invasion upon treatment with varying concentrations of SB 203580 is expressed as average percentage +/- S.E. and represents 3 dishes in each group. The data are typical of triplicate experiments. Figure 1B shows that there is a dose dependent reduction of in-vitro invasion by 43 +/- 9% and 69 +/- 8% using concentrations of 5µM and 10µM, respectively.

Figure 1C shows that the exposure of UM-SCC-1 cells to 5µM and 10µM of the p38 inhibitor for up to 5 days does not affect cell growth excluding antimitogenic effects of the compound to be responsible for the inhibitory effect on in-vitro invasion. p38 isoforms differ with respect to their sensitivity towards SB 203580. The reported IC₅₀ is 0.1 µM for p38alpha but 5-10 µM for p38beta. p38gamma and p38delta are not inhibited by the imidazol derivative (27). Hence, the concentration of SB 203580 required to reduce MMP-9 expression and in-vitro invasion of UM-SCC-1 cells closely matches the IC₅₀ of p38beta. In this experiment the cells were grown in culture medium containing 10% FBS for 5 days with various amounts of SB 203580. Number of viable cells was determined with 0.2 mg/ml MTT-vital stain and consecutive reading of DMSO-dissolved formazan crystals by spectrophotometry at 570 nm on the indicated days. The data points are typical of triplicate experiments.

Thus, experiment 1 and the associated Figure 1 show that the p38 SAPK inhibitor SB 203580 reduces high-level expression of MMP-9 and in-vitro invasion of UM-SCC-1 cells without having any effect on cell growth.

### Experiment 2

In this experiment different cell lines were plated out on filters precoated with Matrigel® in serum-free medium and incubated. Invasion is expressed as the average percentage +/- S.E. and represents 3 dishes in each group. The data are typical of triplicate experiments.

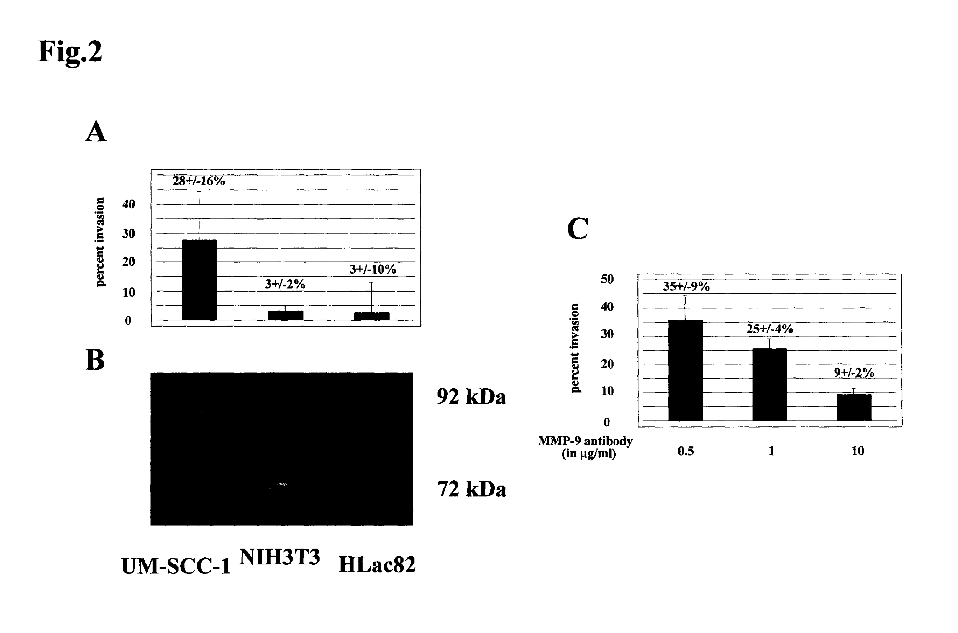

To address the question if MMP-9 might be required for in-vitro invasion of UM-SCC-1 cells, first expression levels of three different cell lines (UM-SCC-1, NIH3T3, Hlac82) were correlated with their in-vitro invasive behavior. Figure 2A shows that the amount of the MMP-9 in the conditioned medium closely paralleled the invasiveness of the cell lines. While UM-SCC-1 cells produce the most protease and exhibit the most invasive phenotype, NIH3T3 cells, which do not secret any detectable MMP-9, were by far less invasive on Matrigel® coated filters. Interestingly, there was no correlation between MMP-2 secretion and invasiveness of the tested cell lines. This might be due to the requirement of the presence of distict membrane type matrix-metalloproteases (MT-MMP's) for the activation of MMP-2 (21), which may not be expressed on NIH3T3 cells.

According to Figure 2B, the different cell lines were changed to serum-free medium and cultured for 48 hours. Condition medium was harvested and cell numbers were determined using MTT. Aliquots of conditioned medium corrected for differences in cell numbers were assayed for MMP-9 activity by zymography as described (20, 25) and as is known by those skilled in the art. The data are typical of triplicate experiments. Only the cell line that expresses the highest amount of MMP-9 shows a clear band in zymography.

To finally demonstrate the requirement for MMP-9 activity for the in-vitro invasive phenotype of UM-SCC-1 cells, in Figure 2C UM-SCC-1 cells were plated out on filters precoated with Matrigel® in serum-free medium and incubated with various amounts of an anti-MMP-9 antibody (0.5, 1, 10µg/ml) or equivalent amounts of preimmun serum provided by the manufacturer for 60 hours. Invasion is expressed as the percentage of cells invading through Matrigel®. Invasion upon treatment with varying concentrations of the antibody recognizing the active and latent form of MMP-9 or preimmune serum provided by the manufacturer is expressed as average percentage +/- S.E. and represents 3 dishes in each group. The data are typical of triplicate experiments. Figure 2C shows that there is a dose dependent reduction of in-vitro invasion with increasing concentrations of the antibody.

Thus, experiment 2 and the associated Figure 2 show that there is a requirement for MMP-9 secretion into the conditioned medium of UM-SCC-1 cells for in-vitro invasion of the cell line. Therefore it can be concluded, that SB 203580 inhibits in-vitro invasion of UM-SCC-1 cells by reducing MMP-9 expression via a p38 signaling pathway.

### Experiment 3

As MMP-9 expression is almost exclusively regulated at the promoter level (28, 29), and to further characterize the role of different p38 isoforms in the regualtion of MMP-9 expression, UM-SCC-1 cells were transiently transfected using Lipofectamin® (Gibco Life Technologies, Karlsruhe, Germany) as described by the manufacturer and a chloramphenicol acetyl transferase (CAT) reporter driven by the wild type MMP-9 promoter or the promoterless CAT construct (SV₀) and the indicated amount (where 2 is a twofold molar excess of the effector plasmid relative to the reporter construct) of an expression vector including a dominant negativ p38alpha, p38beta, p38gamma and p38delta or the empty vector (pCDNA3). Differences in transfected DNA-amount were normalized with empty vector. Cell extracts, normalized for differences in protein amount, were assayed for CAT expression using CAT-ELISA according to the manufacturer (Roche Diagnostics, Mannheim, Germany). Data are expressed as average percent of control +/- S.E. and represent 2 dishes in each group, performed in 3 seperate experiments. The kinase deficient mutants were created by substituting the threonine by alanine and the tyrosine by phenylalanine in the typical TGY sequence of the p38 kinases and all resultant cDNA's cloned into the mammalian expression vector pCDNA3 by standard molecular biology techniques as known by those skilled in the art.

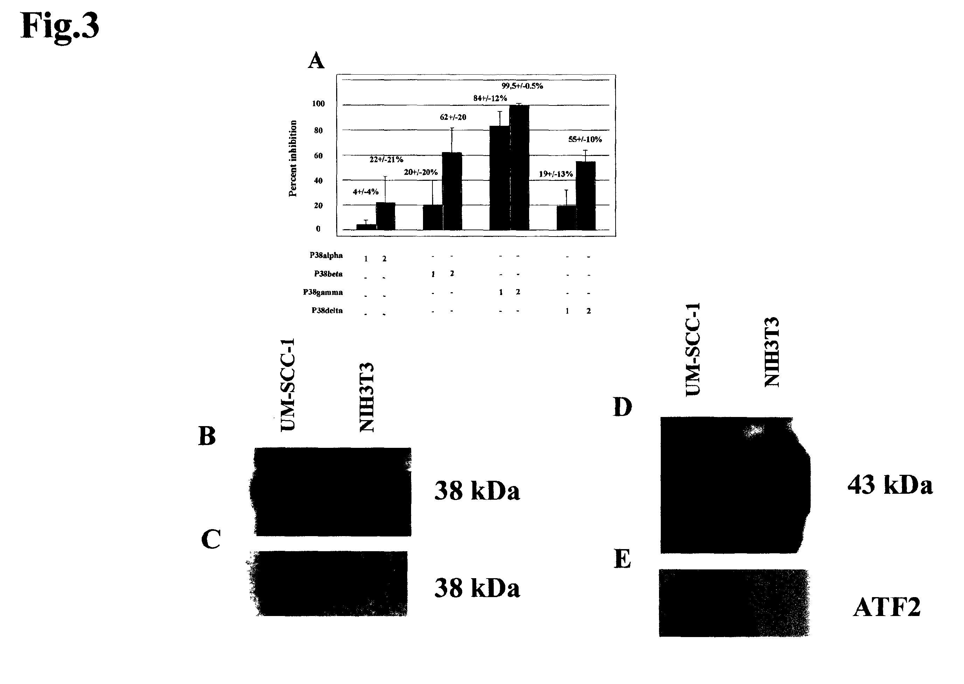

According to Figure 3A, a SB 203580 sensitive isoform mutant, p38beta, was found to repress the activity of the MMP-9 promotor driven CAT reporter by 62 +/- 20% at a twofold molar excess, while, quite in contrast, the p38alpha mutant only reduced MMP-9 promoter activity by 21 +/- 20%. The p38delta mutant inhibited the MMP-9 promoter by 55 +/- 9%. Transfection with the p38gamma mutant virtually silenced the MMP-9 promoter, i. e. promoter activity was repressed by 99.9 +/-0.5%. No significant CAT expression was noted upon transfection of the promoterless CAT construct.

Figure 3A shows that p38gamma besides p38beta and p38delta, but not p38alpha, dominant negative expression constructs reduce MMP-9 promoter activity. This experiment further supports the involvement of p38beta rather than p38alpha in the constitutive activation of the MMP-9 promoter and in addition, they strongly suggest a role for p38delta and most importantly p38gamma in the constitutive activation of the MMP-9 promoter.

In Figure 3B-E UM-SCC-1 and NIH3T3 cells were maintained in culture medium containing 10% FBS. Protein extracts (equal protein) and in-gel kinase assay were prepared as indicated above and subjected to either immunoblotting using a polyclonal anti-p38alpha- or SAPK3- antibody (B) and (D), or in-gel kinase assay using MBP as a substrate (C), or immunokinase reaction using recombinant ATF2 as a substrate (E). The data are typical of triplicate experiments. By these experiments it was excluded that the modest reduction of MMP-9 promoter activity observed after inhibition of p38alpha by a kinases deficient mutant could also be due to missing expression and/or activity of the kinase. For this, UM-SCC-1 cells and NIH3T3 cells (negative control) were assayed for activity and expression of p38alpha. Expression and activity of p38gamma was also anylyzed by immunoprecipitation with a polyclonal SAPK3-antibody and subsequent kinase reaction in a similar experiment.

As can be gathered from Figure 3 B-E presence of both proteins, p38alpha and p38gamma, was noted in both cell lines (Figure 3B and C). However, enzyme activity of both p38 isoforms was found to be high in UM-SCC-1 cells as opposed to undetectable in NIH3T3 cells. Hence, p38alpha and p38gamma are present and constitutively active in UM-SCC-1 cells.

### Experiment 4

Here UM-SCC-1 and NIH3T3 cells were changed to serum-free medium and cultured for 48 hours. Condition medium was harvested and cell numbers were determined using MTT. Aliquots of conditioned medium corrected for differences in cell number were assayed for MMP-9 activity by zymography. The data are typical of triplicate experiments.

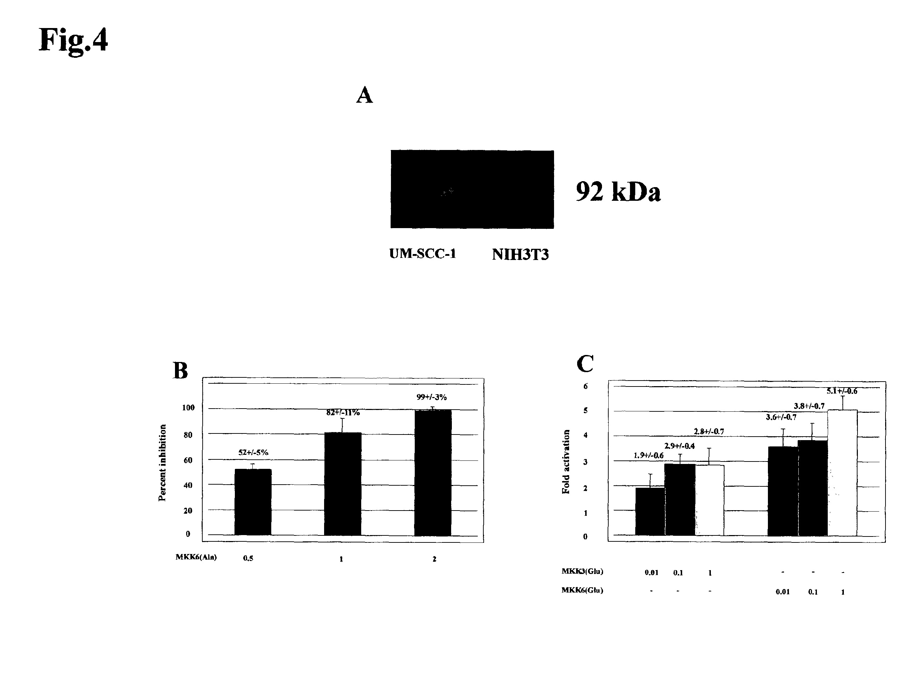

Figure 4A shows that UM-SCC-1 cells, but not NIH3T3 cells, express the matrix-metalloprotease 9 (MMP-9).

As MKK6 protein kinase broadly activates p38 isoforms (in contrast to MKK3, which acts as rather specific activator of the p38alpha and SAPK4/p38delta isoforms) (26, 30), and in order to determine the role of MKK6 in the regulation of MMP-9, in Figure 4B UM-SCC-1 cells were transiently transfected using a CAT reporter driven by the wild type MMP-9 promoter or promoterless CAT construct (SV₀) and the indicated amount (where 2 is a twofold molar excess of the effector plasmid relative to the reporter construct) of an expression vector encoding a kinase dead MKK6 mutant. The kinase dead phenotype was created by substituting serine¹⁵¹ and threonine¹⁵⁵ with alanine according to the above noted publication (26). A strong reduction of MMP-9 promoter activity by 99 +/- 0.5% was observed. These data demonstrate that a MKK6 kinase deficient mutant represses MMP-9 promoter activity in UM-SCC-1 cells and that MKK6 is an upstream regulator of MMP-9, which likely signals through p38 isoforms.

In order to further characterize the role of MKK6 in the regulation of the MMP-9 promoter, the effect of constitutive activation of MKK6 kinase was examined. In Figure 4C NIH3T3 cells were transiently transfected using a CAT reporter driven by the wild type MMP-9 promoter or the promoterless CAT construct (SV₀) and the indicated amount (where 2 is a twofold molar excces of the effector plasmid relative to the reporter construct) of an expression vector encoding a constitutively activated MKK6 and MKK3 protein or the empty vector. Differences in transfected DNA-amount were normalized with empty vector. Cell extracts, normalized for differences in protein amount, were assayed for CAT expression using CAT-ELISA. Data are expressed as average percent of control +/-S.E. and represent 2 dishes in each group, performed in 3 seperate experiments. The constitutive activation of MKK6 was achieved by substituting serine¹⁵¹ and threonine¹⁵⁵ by glutamic acid according to the publication of J. Hahn et al (26). To avoid interfering activation of the promoter by endogenous stimulators, NIH3T3 cells, which do not express endogenous MMP-9 (Figure 4A) were utilized. A five fold activation of the MMP-9 promoter was noted after co-transfection with a CAT reporter and the MKK6 mutant at a one fold molar excess. The same experiment was repeated with a similarly created MKK3 mutant. At a similar molar excess, only a 2.8 fold induction of the promoter was observed.

Figure 4C demonstrates that there is a rather unspecific activation of all p38 isoforms (including p38gamma) by MKK6, while MKK3 more narrowly targets p38alpha and p38delta. These results support the role for p38gamma in the regulation of MMP-9.

All these results clearly demonstrate that according to the present invention cancer, preferably invasiveness of cancer metastasis will be positively influenced by administration of an active agent which influences, particularly inhibits downstream regulators of the MMP-9 signal transduction pathway.

### Literature

1. Simon, C., M.J. Hicks, A.J. Nemechek, R. Meetha, B.W. O'Malley, H. Goepfert, C.M. Flaitz, and D. Boyd. 1999. PD 098059, an inhibitor of ERK1 activation, attenuates the in vivo invasiveness of head and neck squamous cell carcinoma. Br J Cancer 80: 1412-1419
2. Ikebe, T., M. Shinohara, H. Takeuchi, M. Beppu, S. Kurahara, S. Nakamura, and K. Shirasuna. 1999. Gelatinolytic activity of matrix metalloproteinase in tumor tissues correlates with the invasiveness of oral cancer. Clin Exp Metastasis 17:(4) 315-323
3. Denhardt, D.T. 1996. Signal-transducing protein phosphorylation cascades mediated by Ras/Rho proteins in the mammalian cell: the potential for multiplex signaling. Biochem J 318: 729-747
4. Hunter, T. 1997. Oncoprotein networks. Cell 88:(3) 333-346
5. Lengyel, E., R. Gum, E. Stepp, J. Juarez, H. Wang, and D. Boyd. 1996. Regulation of urokinase-type plasminogen activator expression by an ERK1-dependent signaling pathway in a squamous cell carcinoma cell line. J Cell Biochem. 61:(3) 430-443
6. Oka, H., Y. Chatani, R. Hoshino, O. Ogawa, Y. Kakehi, T. Terachi, Y. Okada, M. Kawaichi, M. Kohno, and O. Yoshida. 1995. Constitutive activation of mitogen-activated protein (MAP) kinases in human renal cell carcinoma. Cancer Res 55:(18) 4182-4187
7. Kim, S.C., J.S. Hahn, Y.H. Min, N.C.Yoo, Y.W. Ko, and W.J. Lee. 1999. Constitutive activation of extracellular signal-regulated kinase in human acute leukemias: combined role of activation of MEK, hyperexpression of extracellular signal-regulated kinase, and downregulation of a phosphatase, PAC1. Blood 93:(11) 3893-3899
8. Garrington, T.P. and G.L. Johnson. 1999. Organisation and regulation of mitogen-activated protein kinase signaling pathways. Curr Opin Cell Biol 11:(2) 211-218
9. Lee, J.C., J.T. Laydon, P.C. McDonnell, T.F. Gallagher, S. Kumar, D. Green, D. McNulty, M.J. Blumenthal, J.R. Heys, S.W. Landvatter, J.E. Strickler, M.M. McLaughlin, I.R. Siemens, S.M. Fisher, G.P. Livi, J.R. White, J.L. Adams, and P.R. Young. 1994. A protein kinase involved in the regulation of inflammatory cytokine biosynthesis. Nature 372: 739-746
10. Jiang, Y., C. Chen, Z. Li, W. Guo, J.A. Gegner, S. Lin, and J. Han. 1996. Characterization of the structure and function of a new mitogen-activated protein kinase (p38beta). J Biol Chem 271:(30) 17920-17926
11. Li, Z., Y. Jiang, R.J. Ulevitch, and J. Han. 1996. The primary structure of p38gamma: A new member of p38 group of MAP kinases. Biochem Biophys Res Commun 228:(2) 334-340
12. Wang, X.S., K. Diener, C.L. Manthey, S. Wang, B. Rosenzweig, J. Bray, J. Delaney, C.N. Cole, P. Chan-Hui, N. Mantlo, H.S. Lichenstein, M. Zukowski, and Z. Yao. 1997. Molecular cloning and characterization of a novel p38 mitogen-activated protein kinase. J Biol Chem 272:(38) 23668-23674
13. Lechner, C., M.A. Zahalka, J.F. Giot, N.P. Moller, and A. Ullrich. 1996. ERK6, a mitogen-activated protein kinase involved in C2C12 myoblast differentiation. Proc Natl Acad Sci USA 93:(9) 4355-4359
14. Conrad, P.W., R.T. Rust, J. han, E.E. Millhorn, and D. Beitner-Johnson. 1999. Selective activation of p38alpha and p38gamma by hypoxia. J Biol Chem 274:(33) 23570-23576
15. Mertens, S., M. Craxton, and M. Goedert. 1996. SAP kinase-3, a new member of the family of mammalian stress-activated protein kinases. FEBS Letters 383:(3) 273-276
16. Fang, F.M., S.W. Leung, C.C. Huang, Y.T. Liu, C.J. Wang, H.C. Chen, L.M. Sun, and D.T. Huang. 1997. Combined-modality therapy for squamous carcinoma of the buccal mucosa: treatment results and prognostic factors. Head Neck 19(6) 506-512
17. Kugler, A. 1999. Matrix metalloproteinases and their inhibitors. Anticancer Res 19:(2C) 1589-1592
18. Murray, G.I., M.E. Duncan, E. Arbuckle, W.T. Melvin, and J.E. Fothergill. 1998. Matrix metalloproteinases and their inhibitors in gastric cancer. Gut 43:(6) 791-797
19. Denhardt, D.T., B. Feng, D.R. Edwards, E.T. Cocuzzi, and U.M. Malyankar. 1993. Tissue inhibitor of metalloproteinases (TIMP, aka EPA):; structure, control of expression and biological functions. Pharmacol Ther 59:(3) 329-341
20. Simon, C., H. Goepfert, and D. Boyd. 1998. Inhibition of the p38 mitogen-activated protein kinase by SB 203580 blocks PMA-induced Mr 92,000 type collagenase secretion and in vitro invasion. Cancer Res 58: 1135-1139
21. Price, J.T., M.T. Bonovich, and E.C. Kohn. 1997. The biochemistry of cancer dissemination. Critical Reviews in Biochemistry and Molecular Biology 32:(3) 175-253
22. Fukami, Y., A.A. Tokmakov, K. Konaka, and K. Sato. 1999. Peptide inhibitors of the mitogen-activated protein kinase pathway: a structure -mimetic peptide corresponding to the conserved inter-DFG-APE region in the kinase domain. Pharmacol Ther 82:(2-3) 399-407
23. Lee, J.C., S. Kassis, S. Kumar, A. Badger, and J.L. Adams. (1999). p38 mitogen-activated protein kinase inhibitors-mechanisms and therapeutic potentials. Pharmacol Ther 82:(2-3) 389-397
24. Fabbro, D., E. Buchdunger, J. Wood, J. Mestan, F. Hofman, S. Ferrari, H. Mett, T. O'Reilly, and T. Meyer. (1999). Inhibitors of protein kinases: CGP 41251, a protein kinase inhibitor with potential as an anticancer agent. Pharmacol Ther 82:(2-3) 293-301
25. Simon, C., J. Juarez, G.L. Nicolson, and D. Boyd. 1996. 1996. Effect of PD 098059, a specific inhibitor of mitogen-activated protein kinase kinase, on urokinase expression and in vitro invasion. Cancer Res 56: 5369-5374
26. Han, J., J.D. Lee, Y. Jiang, Z. Li, L. Feng, and R.J. Ulevitch. 1996. Characterization of the structure and function of a novel MAP kinase kinase (MKK6). J Biol Chem 271:[6) 2886-2891
27. Kumar, S., P.C. McDonnell, R.J. Gum, A.T. Hand, J.C. Lee, and P.R. Young. 1997. Novel homologues of CSBP/p38 MAP kinase: Activation, substrate specificity and sensitivity to inhibition by pyridinyl imidazoles. Biochem Biophys Res Commun 235:(3) 533-538
28. Gum, R., H. Wang, E. Lengyel, J. Juarez, and D. Boyd. 1997. Regulation of 92 kDa type 4 collagenase expression by the jun aminoterminal kinase- and the extracellular signal-regulated kinase-dependent signaling cascades. Oncogene 14: 1481-1493
29. Gum, R., E. Lengyel, J. Juarez, J.H. Chen, H. Sato, M. Seiki, and D. Boyd. 1996. Stimulation of 92-kDa gelatinase B promoter activity by ras is mitogen-activated protein kinase kinase-1-independent and requires multiple transcription factor binding sites including closely spaced PEA3/ets and AP-1 sequences. J Biol Chem 271:(18) 10672-10680
30. Cuenda, A., P. Cohen, V. Buee-Scherrer, and M. Goedert. 1996. Activation of stress-activated protein kinase-3 (SAPK3) by cytokines and cellular stresses is mediated via SAPKK3 (MKK6): comparison of the specificities of SAPK3 and SAPK2 (RK/p38). EMBO J 16:(2) 295-305

## Claims

1. Use of an active agent for influencing, particularly inhibiting the expression of matrix-metalloproteases in eukaryotic cells, for the preparation of a medicament or a pharmaceutical composition for the treatment of cancer.

2. Method for the treatment of cancer, **characterized in that** eukaryotic cells are treated by an active agent which influences, particularly inhibits the expression of matrix-metalloproteases.

3. Use or method according to claim 1 or 2, **characterized in that** said matrix-metalloprotease is the matrix-metalloprotease-9 (MMP-9).

4. Use or method according to one of the preceding claims, **characterized in that** said active agent is targeted against at least one member of the matrix-metalloprotease signal transduction pathway.

5. Use or method according to claim 4, **characterized in that** said member is a member of the p38 protein family.

6. Use or method according to claim 5, **characterized in that** said p38 protein is the p38beta protein.

7. Use or method according to claim 5, **characterized in that** said p38 protein is the p38gamma (SAPK3 or ERK6) protein.

8. Use or method according to claim 4, **characterized in that** said member is a member of the mitogen-activated kinase kinase family.

9. Use or method according to claim 8, **characterized in that** said mitogen-activated kinase kinase is the mitogen-activated kinase kinase 6 (MKK6) or the mitogen-activated kinase kinase 3 (MKK3).

10. Use or method according to one of the preceding claims, **characterized in that** said active agent is targeted against activators, regulators and/or biological precursors of the matrix-metalloprotease signal transduction pathway.

11. Use or method according to one of the preceding claims, **characterized in that** said active agent is a small molecular compound, preferably a small molecular compound with a molecular weight (MW) < 1000.

12. Use or method according to claim 11, **characterized in that** the small molecular compound is an imidazole derivative, wherein preferably said imidazole derivative is SB 203580 or SB 202190.

13. Use or method according to one of the preceding claims, **characterized in that** said active agent is a polynucleotide encoding a peptide, preferably a polypeptide, which influences, preferably inhibits the expression of matrix-metalloproteases.

14. Use or method according to one of the preceding claims, **characterized in that** said cancer is of the invasive phenotype.

15. Use or method according to one of the preceding claims, **characterized in that** said cancer is;
a) a squamous epithelial carcinoma, preferably a squamous epithelial carcinoma of the head, neck, skin or stomach, or
b) a colon-, breast- or hepatocellular carcinoma, or
c) a fibrosarcoma of the stomach.

16. Pharmaceutical composition, comprising a compatible quantity of at least one active agent, wherein said active agent is influencing, preferably inhibiting the expression of matrix-metalloproteases in eukaryotic cells, and optionally further comprising a pharmaceutically acceptable carrier.

17. Pharmaceutical composition according to claim 16, further **characterized by** an active agent as defined in one of the claims 4 to 13.
